# EUROPEAN PATENT APPLICATION

(11) **EP 2 527 458 A2**
(43) Date of publication of application: **28.11.2012**
(21) Application number: 12180644.2
(22) Date of filing: 02.05.2007
(51) Int. Cl.: C12Q 1/48, C12Q 1/32, G01N 33/50, G01N 33/68

(54) **Methods and reagents for detecting susceptilbility to graft versus host disease**

(30) Priority: 02.05.2006 US 797037 P
(62) Divisional of application: 07794518.6
(71) Applicant: THERAKOS, INC., Exton, PA 19341 (US)
(72) Inventor: O'Brien, Peter, J, Downingtown, PA Pennsylvania 19335 (US)
(74) Representative: Carpmaels & Ransford

(57) **Abstract**

The present invention provides methods for determining the likelihood and/or severity of GvHD and the likelihood of the occurrence of transplant related mortality.

## Description

### STATEMENT REGARDING FEDERALLY SPONSORED RESEARCH OR DEVELOPMENT

No government funds were used to make this invention.

### BACKGROUND OF THE INVENTION

Graft versus host disease (GVHD) occurs in the context of transplantation. In GVHD donor T-cells reject recipient's tissues and organs by mounting an attack against the recipient's body. A host of other diseases involve disregulation of the host immune system. Some are best treated with pharmaceuticals, some with biologicals, others with treatments such as extracorporeal photophoresis, and yet others have very limited treatment options.

Extracorporeal photopheresis (ECP) has been shown to be an effective therapy in certain T-cell mediated diseases. In the case of GVHD, photopheresis has been used as a treatment in association with topical triamcinolone ointments, antifungal, antiviral, antibiotics, immunoglobulins, and methotrexate. ECP has also been used with immunosuppressive agents such as mycophenolate mofetil, tacrolimus, prednisone, cyclosporine, hydroxychloroquine, steroids, FK-506, and thalidomide for cGVHD and refractory cGVHD. For solid organ transplants, ECP has been used in conjunction with immunosuppressive agents to reduce the number of acute allograft rejection episodes associated with renal allografts and cardiac transplants. For example, ECP has been used with OKT3 and/or the immunosuppressive agents prednisone, azathioprine, and cyclosporine to reverse acute renal allograft rejection. ECP has also been used with cyclophosphamide, fractionated total body irradiation, and etoposide for allogeneic marrow transplantation for acute myeloid leukemia, acute lymphoblastic leukemia, chronic myeloid leukemia, non-Hodgkin's lymphoma, or severe aplastic anemia.

### SUMMARY OF THE INVENTION

Several experimental strategies have identified pharmacodynamic biomarkers for ECP. Flow cytometric analysis of patient samples in the pGvHD trial indicate that the relative abundance of certain dendritic cell subsets are predictive of the propensity to develop GvHD and also reflect the impact of ECP on that process.

Correlation of the flow cytometric data with central lab data led to additional candidate biomarkers that correlate with the DC ratio and therefore obviate the need for flow cytometric studies. In addition, a subset of B cells was identified that correlates with the DC subsets under study. The present invention provides a set of markers that are readily measured and, when analyzed together, provide a statistically robust measure of ECP action and disease progression.

### BRIEF DESCRIPTION OF THE DRAWINGS

Figure 1 shows the clinical trial and study design.
Figure 2 shows cytometric phenotyping of PBMC.
Figure 3 shows that DC profiles predict severe GvHD. DC Subsets vary with GvHD Grade. Heat Map of DC abundance which, along with the plot of CD11 c+/CD11 cDC shown in (B.) demonstrate the difference in relative expression of these DC subtypes in patients that later suffered GvHD. (C.) and (D.) show the results of logistic regression analysis of cell surface markers. Here, the ROC reflects the utility of the marker for discriminating between grade 0 or 1 and severe GvHD populations. A better test has an ROC closer to 1, the worst test ROC is 0.5.
Figure 4 shows that DC profiles predict TRM.
Figure 5 shows modeling with different biomarkers and shows that the best predictor of TRM found in this study is a model including CD11c+ DC and NK cells.

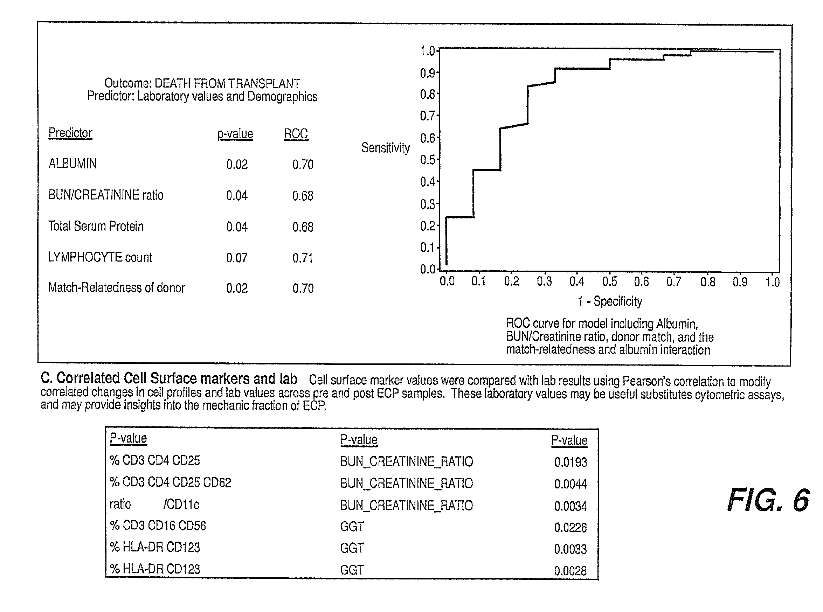

Figure 6 shows A) Models predicting GvHD. with 59 results, only monocyte-(ROC=0.66) and neutrophil counts (ROC=0.69) approached the predictive power of DC subsets for GvHD. B) Models predicting TRM. Combinations of lab values and patient demographics yielded models with remarkable predictive power for TRM. Results are shown independently (below left) and when modeled in combination (ROC curve below right). The ROC curve for the model included albumin, BUN/creatinine ratio, donor match, and the match-relatedness and albumin interaction. C) Correlated cell surface markers and Lab values. Cell surface marker values were compared with lab results using Pearson's correlation to identify correlated changes in cell profiles and cytometric assays.

### DETAILED DESCRIPTION OF THE INVENTION

All references cited in this Description are incorporated in this specification in their entirety. The terms "subject" or "patient" are used interchangeably and refer to an animal, preferably a mammal and more preferably a human.

A "cell population" generally includes a cell type found in blood. The term may include one or more types of blood cells, specifically, red blood cells, platelets, and white blood cells. A cell population may comprise subtypes of white blood cells, for example, T-cells, dendritic cells, B-cells, etc. In one embodiment, a cell population may comprise a mixture or pool of cell types. Alternatively, a cell population may comprise a substantially purified type of cells, for example, T-cells or dendritic cells.

"ECP procedure" or "ECP" refers to extracorporeal photopheresis, also known as extracorporeal phototherapy. It is a treatment of a population of cells that has been subjected to UVA light and a photoactivable compound. Preferably the population of cells is from an organ or tissue; more preferably, the population of cells is a portion of blood; and most preferably, the population of cells is a buffy coat. ECP is sometimes used to refer to a process in which a cell population has been subjected to an apoptosis-inducing procedure with UVA light in the presence of a DNA cross linking agent such as a psoralen (preferably, 8-MOP).

In the most preferred embodiment of the invention, ECP is used to induce apoptosis. This involves a photoactivatable compound added to a cell population ex vivo. The photosensitive compound may be administered to a cell population comprising blood cells following its withdrawal from the subject, recipient, or donor, as the case may be, and prior to or contemporaneously with exposure to ultraviolet light. The photosensitive compound may be administered to a cell population comprising whole blood or a fraction thereof provided that the target blood cells or blood components receive the photosensitive compound. In another embodiment, a portion of the subject's blood, recipient's blood, or the donor's blood could first be processed using known methods to substantially remove the erythrocytes and the photoactive compound may then be administered to the resulting cell population comprising the enriched leukocyte fraction.

In an alternative embodiment, the photoactivatable compound may be administered in vivo. The photosensitive compound, when administered to a cell population comprising the subject's blood, recipient's blood, or the donor's blood, as the case may be, in vivo may be administered orally, but also may be administered intravenously and/or by other conventional administration routes. The oral dosage of the photosensitive compound may be in the range of about 0.3 to about 0.7 mg/kg, more specifically, about 0.6 mg/kg. When administered orally, the photosensitive compound may be administered at least about one hour prior to the photopheresis treatment and no more than about three hours prior to the photopheresis treatment.

Photoactivatable compounds for use in accordance with the present invention include, but are not limited to, compounds known as psoralens (or furocoumarins) as well as psoralen derivatives such as those described in, for example, U.S. Pat. No. 4,321,919 and U.S. Pat. No. 5,399,719. Preferred compounds include 8-methoxypsoralen; 4,5'8-trimethylpsoralen; 5-methoxypsoralen; 4-methylpsoralen; 4,4-dimethylpsoralen; 4-5'-dimethylpsoralen; 4'-aminomethyl-4,5',8-trimet- hylpsoralen; 4'-hydroxymethyl-4,5',8-trimethylpsoralen_{'}, 4',8-methoxypsoralen; and a 4'-(omega-amino-2-oxa) alkyl-4,5'8-trimethylpsoralen, including but not limited to-4'-(4-amino-2-oxa)butyl-4,5',8-trimethylpsoralen. In one embodiment, the photosensitive compound that may be used comprises the psoralen derivative, amotosalen (S-59) (Cerus, Corp., Concord, Calif.). In another embodiment, the photosensitive compound comprises 8-methoxypsoralen (8 MOP).

The cell population to which the photoactivatable compound has been added is treated with a light of a wavelength that activates the photoactivatable compound. The treatment step that activates the photoactivatable compound is preferably carried out using long wavelength ultraviolet light (UVA), for example, at a wavelength within the range of 320 to 400 nm. The exposure to ultraviolet light during the photopheresis treatment preferably is administered for a sufficient length of time to deliver about 1-2 J/cm.sup.2 to the cell population.

Extracorporeal photopheresis apparatus useful in the methods according to the invention include those manufactured by Therakos, Inc., (Exton, Pa.) under the name UVAR™ A description of such an apparatus is found in U.S. Pat. No. 4,683,889. The UVAR™ System uses a treatment system and consists of three phases including: 1) the collection of a buffy-coat fraction (leukocyte-enriched), 2) irradiation of the collected buffy coat fraction, and 3) reinfusion of the treated white blood cells. The collection phase has six cycles of blood withdrawal, centrifugation, and reinfusion steps. During each cycle, whole blood is centrifuged and separated in a pheresis bowl. From this separation, plasma (volume in each cycle is determined by the UVAR™. Instrument operator) and 40 ml buffy coat are saved in each collection cycle. The red cells and all additional plasma are reinfused to the patient before beginning the next collection cycle. Finally, a total of 240 ml of buffy coat and 300 ml of plasma are separated and saved for UVA irradiation.

The irradiation of the leukocyte-enriched blood within the irradiation circuit begins during the buffy coat collection of the first collection cycle. The collected plasma and buffy coat are mixed with 200 ml of heparinized normal saline and 200 mg of UVADEX™ (water soluble 8-methoxypsoralin). This mixture flows in a 1.4 mm thick layer through the PHOTOCEPTOR™ Photoactivation Chamber, which is inserted between two banks of UVA lamps of the PHOTOSETTE™ PHOTOSETTE™ UVA lamps irradiate both sides of this UVA-transparent PHOTOCEPTOR™ chamber, permitting a 180-minute exposure to ultraviolet A light, yielding an average exposure per lymphocyte of 1-2 J/cm². The final buffy coat preparation contains an estimated 20% to 25% of the total peripheral blood mononuclear cell component and has a hematocrit from.2.5% to 7%. Following the photoactivation period, the volume is reinfused to the patient over a 30 to 45 minute period. U.S. patent application Ser. No. 09/480,893 (incorporated herein by reference) describes another system for use in ECP administration. U.S. Pat. Nos. 5,951,509; 5,985,914; 5,984,887,4,464,166; 4,428,744; 4,398,906; 4,321,919; PCT Publication Nos. WO 97/36634; and WO 97/36581 also contain description of devices and methods useful in this regard.

Another system that may be useful in the methods of the present invention is described in U.S. patent application Ser. No. 09/556,832. That system includes an apparatus by which the net fluid volume collected or removed from a subject may be reduced during ECP. The effective amount of light energy that is delivered to a cell population may be determined using the methods and systems described in U.S. Pat. No. 6,219,584.

A variety of other methods for inducing apoptosis in a cell population are well-known and may be adopted for use in the present invention. One such treatment comprises subjecting a cell population to ionizing radiation (gamma-rays, x-rays, etc.) and/or non-ionizing electromagnetic radiation including ultraviolet light, heating, cooling, serum deprivation, growth factor deprivation, acidifying, diluting, alkalizing, ionic strength change, serum deprivation, irradiating, or a combination thereof. Alternatively, apoptosis may be induced by subjecting a cell population to ultrasound.

Yet another method of inducing apoptosis comprises the extracorporeal application of oxidative stress to a cell population. This may be achieved by treating the cell population, in suspension, with chemical oxidizing agents such as hydrogen peroxide, other peroxides and hydroperoxides, ozone, permanganates, periodates, and the like. Biologically acceptable oxidizing agents may be used to reduce potential problems associated with residues and contaminations of the apoptosis-induced cell population so formed.

In preparing the apoptosis-induced cell population, care should be taken not to apply excessive levels of oxidative stress, radiation, drug treatment, etc., because otherwise there may be a significant risk of causing necrosis of at least some of the cells under treatment. Necrosis causes cell membrane rupture and the release of cellular contents often with biologically harmful results, particularly inflammatory events, so that the presence of necrotic cells and their components along with the cell population comprising apoptotic cells is best avoided. Appropriate levels of treatment of the cell population to induce apoptosis, and the type of treatment chosen to induce apoptosis are readily determinable by those skilled in the art.

One process according to the present invention involves the culture of cells from the subject, or a compatible mammalian cell line. The cultured cells may then be treated extracorporeally to induce apoptosis and to create a cell population therein. The extracorporeal treatment may be selected from the group consisting of antibodies, chemotherapeutic agents, radiation, extracorporeal photopheresis, ultrasound, proteins, and oxidizing agents. The cells, suspended in the subject's plasma or another suitable suspension medium, such as saline or a balanced mammalian cell culture medium, may then be administered to the patient.

Methods for the detection and quantitation of apoptosis are useful for determining the presence and level of apoptosis in the preparation to be administered to the subject in the present invention. The number of apoptotic cells in a cell population required to obtain the required clinical benefit in a subject may vary depending on the source of cells, the subject's condition, the age and weight of the subject and other relevant factors, which are readily determinable by well-known methods. Preferably, the number of apoptotic cells that are administered to a patient are 0.1 to 50 billion, more preferably 1 to 10, and most preferably 2.5 to 7.5 billion.

In one embodiment, cells undergoing apoptosis may be identified by a characteristic 'laddering' of DNA seen on agarose gel electrophoresis, resulting from cleavage of DNA into a series of fragments. In another embodiment, the surface expression of phosphatidylserine on cells may be used to identify and/or quantify an apoptosis-induced cell population. Measurement of changes in mitochondrial membrane potential, reflecting changes in mitochondrial membrane permeability, is another recognized method of identification of a cell population. A number of other methods of identification of cells undergoing apoptosis and of a cell population, many using monoclonal antibodies against specific markers for a cell population, have also been described in the scientific literature.

Acute solid organ transplantation rejection occurs in 30% to 60% of patients after lung transplantation and to a lower degree with liver, kidney, heart etc. due to the success of immunosuppressive agents. The lymphocyte (cell)-mediated immune reaction against transplantation antigens, is the principal mechanism of acute rejection. A delayed or chronic rejection causes graft destruction in months to years after transplantation and is characterized by vascular destruction leading to necrosis of the transplanted tissue. This rejection is not currently suppressed to any large degree by standard regimens and thus the need for more sustainable immune tolerance is a significant unmet need.

Late graft deterioration occurs occasionally, and this chronic type of rejection often progresses insidiously despite increased immunosuppressive therapy. The pathologic picture differs from that of acute rejection. The arterial endothelium is primarily involved, with extensive proliferation that may gradually occlude the vessel lumen, resulting in ischemia and fibrosis of the graft.

Immunosuppressants are currently widely used to control the rejection reaction and are primarily responsible for the success of transplantation. However, these drugs suppress all immunologic reactions, thus making overwhelming infection the leading cause of death in transplant recipients.

Existing immunosuppressant treatment can differ in the case of different types of transplants. Liver allografts are less aggressively rejected than other organ allografts. For example, hyperacute rejection of a liver transplant does not occur invariably in patients who were presensitized to HLA antigens or ABO incompatibilities. Typical immunosuppressive therapy in an adult involves using cyclosporine, usually given IV at 4 to 6 mg/kg/day starting at the time of transplantation and then 8 to 14 mg/kg/day po when feeding is tolerated. Doses are adjusted downward if renal dysfunction occurs, and blood levels are used as approximate measures of adequate dosage.

In heart transplantation, immunosuppressive regimens are similar to those for kidney or liver transplantation. However, in lung and heart-lung transplants acute rejection occurs in >80% of patients but may be successfully managed. Patients are treated with corticosteroids, given rapidly IV in high dosage, ATG, or OKT3. Prophylactic ALG or OKT3 is also frequently given during the first two post-transplant weeks. Pancreas transplantation is unique among the vascularized organ transplants: Instead of being used to save a life, it attempts to stabilize or prevent the devastating target organ complications of type I diabetes. Because the recipient exchanges the risks of insulin injection with the risks of immunosuppression, pancreas transplantation has been generally limited primarily to patients who already need to receive immunosuppressive drugs (i.e., diabetics with renal failure who are receiving a kidney transplant).

Patients with acute myeloid or lymphoblastic leukemia may benefit from bone marrow transplant (BMT). Pediatric BMT has expanded because of its potential for curing children with genetic diseases (e.g., thalassemia, sickle cell anemia, immunodeficiencies, inborn errors of metabolism). Another option for BMT is autologous transplantation (removal of a patient's own marrow when a complete remission has been induced, followed by ablative treatment of the patient with the hope of destruction of any residual tumor and rescue with the patient's own bone marrow). Since an autograft is used, no immunosuppression is necessary other than the short-term high-dose chemotherapy used for tumor eradication and bone marrow ablation; posttransplant problems with GVHD are minimal.

The rejection rate is <5% in transplants for leukemia patients from HLA-identical donors. For multiply transfused patients with aplastic anemia, the rejection rate has also been significantly decreased because of increased immunosuppression during transplant induction. Nonetheless, complications can arise including rejection by the host of the marrow graft, acute GVHD, and infections. Later complications include chronic GVHD, prolonged immunodeficiency, and disease recurrence.

The methods of the present invention can also be used in implant surgery, for example, with implant surgery commonly performed in cosmetic or non-cosmetic plastic surgery. Such implants may include dental, fat grafting, for example to the cheeks, lips and buttocks, facial implants, including those to the nose, cheeks, forehead, chin and skull, buttocks implants, breast implants, etc. Other implants include, but are not limited to, corneal ring, cortical, orbital, cochlear, muscle (all muscles, including pectoral, gluteal, abdominal, gastrocnemius, soleus, bicep, tricep), alloplastic joint and bone replacement, vertebral hair, fetal or stem cell implantation.

The hematopoietic development of dendritic cells (DCs), potent antigen presenting cells (APCs), is distinct and may follow several precursor pathways, some closely linked to monocytes. DCs may be derived from a lymphoid precursor. Thomas et al. (1993) J. Immunol. 150:821 834. Like in blood, there may be three distinct subsets of DCs .present in the thymus: 1) plasmacytoid CD4+ CD11c-DCs; 2) CD4+ CD11c+DCs and 3) interdigitating DCs.

Generation of large numbers of DCs for potential clinical use has recently been accomplished through the in vitro culturing of progenitors with cytokines. Various strategies have been adopted to introduce antigens into dendritic cells so that they may be more effectively presented to T cells in the context of costimulation. It has also been shown that dendritic cells can influence the T cell response to antigen to follow either a humoral or systemic pathway.

DCs are APC that are essential for initiation of primary immune responses and the development of tolerance. DCs express MHC, necessary for stimulation of naive T cell populations. The hematopoietic development of DCs is distinct and may follow several precursor pathways, some of which are closely linked to monocytes. See, for review, Avigan (1999) Blood Rev. 13:51 64. Different DC subsets have distinct developmental pathways. The emerging concept is that one DC subset has regulatory functions that may contribute to the induction of tolerance to self-antigens. Austyn (1998) Curr. Opin. Hematol. 5:3-15.

Studies on DC's in blood were hampered by scarcity of the cells and the relative lack of DC-specific cell surface markers. Methods for DC isolation are based on either maturational change after a short culture period, like the acquisition of low buoyant density or the expression of DC activation/maturation antigens (CD83, CMRF-44 and CMRF-56). Young et al. (1988) Cell Immunol. 111:167; and Van Voorhis et al. (1982) J. Exp, Med. 155:1172.

Isolation of DCs from blood previously relied on a multitude of immunophenotypic criteria, like the absence of a panel of leukocyte lineage (lin)-specific antigens (e.g. CD3, CD14, CD19 and CD56) and the presence of HLA-DR, CD4 or CD33. Romani et al. (1996) J. Immunol. Met. 196:137 151.

From analyses of DC isolated from non-cultured blood it became evident that blood DC are not a homogeneous cell population but a mixture of at least two populations. Thomas et al. (1994). The first blood DC subpopulation is CD123^{bright} CD11c⁻ DC, which possesses a plasmacytoid morphology and potent T cell stimulatory function. The second blood DC subpopulation is CD123^{dim} CD11c^{bright}, which is rather monocytoid in appearance, expresses CD45RO and spontaneously develops into typical mature DCs even when cultured without any exogenous cytokines. Plasmacytoid CD123^{bright} CD11c⁻ DC display some features, like the expression of the pre-T cell receptor a chain, which indicate that they may arise from lymphoid precursors. Bruno et al. (1997) J. Exp. Med. 185:875 884. CD123^{dim} CD11c^{bright} DC display all the criteria of myeloid DCs. DCs resembling plasmacytoid CD123^{brght}CD11c⁻ DC have been detected in the T cell-rich areas of lymphoid tissue and were initially erroneously designated plasmacytoid T cells or plasmacytoid monocytes due to their morphology and phenotype. Grouard et al. (1997) J. Exp. Med. 185:1101 111.

We have now found a DC subset and method of analysis thereof as a method of detecting susceptibility to GVHD, progression and treatment of GVHD and as a biomarker for ECP.

Prevention of GvHD and clinical outcome (at least partly as a result of ECP) are reflected in the peripheral blood CD11c+ to CD11c- ratio and its change after ECP.

As demonstrated by its identification in a clinical trial, this biomarker can be measured reliably with little sophistication required by the physician beyond phlebotomy. Nevertheless, flow cytometric measurement is a sophisticated technique, and requires more technical expertise than is desirable in most applications. The rich data set derived from the pGvHD trial has created several opportunities to identify additional biomarkers that correlate with the presence of this DC subset and that are more readily measured (see below).

A Biomarker is any indicia of the level of expression of an indicated Marker gene. The indicia can be direct or indirect and measure over- or under-expression of the gene given the physiologic parameters and in comparison to an internal control, normal tissue or another carcinoma. Biomarkers include, without limitation, nucleic acids (both over and under-expression and direct and indirect). Using nucleic acids as Biomarkers can include any method known in the art including, without limitation, measuring DNA amplification, RNA, micro RNA, loss of heterozygosity (LOH), single nucleotide polymorphisms (SNPs, Brookes (1999)), microsatellite DNA, DNA hypo- or hyper-methylation. Using proteins as Biomarkers includes any method known in the art including, without limitation, measuring amount, activity, modifications such as glycosylation, phosphorylation, ADP-ribosylation, ubiquitination, etc., or imunohistochemistry (IHC). Other Biomarkers include imaging, cell count and apoptosis Markers.

A Marker nucleic acid corresponds to the sequence designated by a SEQ ID NO when it contains that sequence. A gene segment or fragment corresponds to the sequence of such gene when it contains a portion of the referenced sequence or its complement sufficient to distinguish it as being the sequence of the gene. A gene expression product corresponds to such sequence when its RNA, mRNA, miRNA or cDNA hybridizes to the composition having such sequence (e.g. a probe) or, in the case of a peptide or protein, it is encoded by such mRNA. A segment or fragment of a gene expression product corresponds to the sequence of such gene or gene expression product when it contains a portion of the referenced gene expression product or its complement sufficient to distinguish it as being the sequence of the gene or gene expression product.

The inventive methods, compositions, articles, and kits of described and claimed in this specification include one or more Marker genes. "Marker" or "Marker gene" is used throughout this specification to refer to genes and gene expression products that correspond with any gene the over- or under-expression of which is associated with a likelihood of the occurrence of GvHD or TRM.

The present invention further provides microarrays or gene chips for performing the methods described herein.

The present invention further provides diagnostic/prognostic portfolios containing reagents suitable for measuring Biomarkers such as isolated nucleic acid sequences, their complements, or portions thereof of a combination of genes as described herein where the combination is sufficient to measure or characterize gene.expression in a biological sample.

Any method described in the present invention can further include measuring expression of at least one gene constitutively expressed in the sample.

The invention further provides a method for providing direction of therapy by determining the likelihood of GvHD or TRM according to the methods described herein and identifying the appropriate treatment therefor.

The invention further provides a method for providing a prognosis by determining the likelihood of GvHD or TRM according to the methods described herein and identifying the corresponding prognosis therefor.

The invention further provides a method for finding Biomarkers comprising determining the expression level of a Marker gene, measuring a Biomarker for the Marker gene to determine expression thereof, analyzing the expression of the Marker gene according to the methods described herein and determining if the Marker gene is effectively specific for GvHD or TRM.

The invention further provides kits, articles, microarrays or gene chip, diagnostic/prognostic portfolios for conducting the assays described herein and patient reports for reporting the results obtained by the present methods.

The mere presence or absence of particular nucleic acid sequences in a tissue sample has only rarely been found to have diagnostic or prognostic value. Information about the expression of various proteins, peptides or mRNA, on the other hand, is increasingly viewed as important. The mere presence of nucleic acid sequences having the potential to express proteins, peptides, or mRNA (such sequences referred to as "genes") within the genome by itself is not determinative of whether a protein, peptide, or mRNA is expressed in a given cell. Whether or not a given gene capable of expressing proteins, peptides, or mRNA does so and to what extent such expression occurs, if at all, is determined by a variety of complex factors. Irrespective of difficulties in understanding and assessing these factors, assaying gene expression can provide useful information about the occurrence of important events such as GvHD or TRM, and other clinically relevant phenomena. Relative indications of the degree to which genes are active or inactive can be found in gene expression profiles.

Preferred methods for establishing gene expression profiles include determining the amount of RNA that is produced by a gene that can code for a protein or peptide. This is accomplished by reverse transcriptase PCR (RT-PCR), competitive RT-PCR, real time RT-PCR, differential display RT-PCR, Northern Blot analysis and other related tests. While it is possible to conduct these techniques using individual PCR reactions, it is best to amplify complementary DNA (cDNA) or complementary RNA (cRNA) produced from mRNA and analyze it via microarray. A number of different array configurations and methods for their production are known to those of skill in the art and are described in for instance, 5445934; 5532128; 5556752; 5242974; 5384261; 5405783; 5412087; 5424186; 5429807; 5436327; 5472672; 5527681; 5529756; 5545531; 5554501; 5561071; 5571639; 5593839; 5599695; 5624711; 5658734; and 5700637.

Microarray technology allows for measuring the steady-state mRNA or miRNA level of thousands of genes simultaneously providing a powerful tool for identifying effects such as the onset, or modulation of GvHD. Two microarray technologies are currently in wide use, cDNA and oligonucleotide arrays. Although differences exist in the construction of these chips, essentially all downstream data analysis and output are the same. The product of these analyses are typically measurements of the intensity of the signal received from a labeled probe used to detect a cDNA sequence from the sample that hybridizes to a nucleic acid sequence at a known location on the microarray. Typically, the intensity of the signal is proportional to the quantity of cDNA, and thus mRNA or miRNA, expressed in the sample cells. A large number of such techniques are available and useful. Preferred methods can be found in 6271002; 6218122; 6218114; 6004755; and Keene et al. (2006) RIP-Chip: the isolation and identification of mRNAs, microRNAs and protein components of ribonucleoprotein complexes from cell extracts Nature Protocols 1:302-307.

Analysis of the expression levels is conducted by comparing such signal intensities. This is best done by generating a ratio matrix of the expression intensities of genes in a test sample versus those in a control sample. For instance, the gene expression intensities from a diseased tissue can be compared with the expression intensities generated from normal tissue of the same type. A ratio of these expression intensities indicates the fold-change in gene expression between the test and control samples.

The selection can be based on statistical tests that produce ranked lists related to the evidence of significance for each gene's differential expression between factors related to GvHD or TRM. Examples of such tests include ANOVA and Kruskal-Wallis. The rankings can be used as weightings in a model designed to interpret the summation of such weights, up to a cutoff, as the preponderance of evidence in favor of one class over another. Previous evidence as described in the literature may also be used to adjust the weightings.

Gene expression profiles can be displayed in a number of ways. The most common is to arrange raw fluorescence intensities or ratio matrix into a graphical dendogram where columns indicate test samples and rows indicate genes. The data are arranged so genes that have similar expression profiles are proximal to each other. The expression ratio for each gene is visualized as a color. For example, a ratio less than one (down-regulation) appears in the blue portion of the spectrum while a ratio greater than one (up-regulation) appears in the red portion of the spectrum. Commercially available computer software programs are available to display such data including "GeneSpring" (Silicon Genetics, Inc.) and "Discovery" and "Infer" (Partek, Inc.)

In the case of measuring protein levels to determine gene expression, any method known in the art is suitable provided it results in adequate specificity and sensitivity. For example, protein levels can be measured by binding to an antibody or antibody fragment specific for the protein and measuring the amount of antibody-bound protein. Antibodies can be labeled by radioactive, fluorescent or other detectable reagents to facilitate detection. Methods of detection include, without limitation, enzyme-linked immunosorbent assay (ELISA) and immunoblot techniques.

The gene expression profiles of this invention can also be used in conjunction with other non-genetic diagnostic methods useful in diagnosis, prognosis, or treatment monitoring. For example, in some circumstances it is beneficial to combine the diagnostic power of the gene expression based methods described above with data from conventional Markers such as serum protein Markers. In one such method, blood is periodically taken from a patient and then subjected to an enzyme immunoassay for a serum Markers such as albumin. When the concentration of the Marker suggests the likelihood of GvHD or TRM, a sample source amenable to gene expression analysis is taken. This approach can be particularly useful when other testing produces ambiguous results.

Kits made according to the invention include formatted assays for determining the Biomarker expression. These can include all or some of the materials needed to conduct the assays such as reagents and instructions and a medium through which Biomarkers are assayed.

Articles of this invention include representations of the Biomarker expression useful for treating, diagnosing, prognosticating, and otherwise assessing diseases. These profile representations are reduced to a medium that can be automatically read by a machine such as computer readable media (magnetic, optical, and the like).. The articles can also include instructions for assessing the gene expression profiles in such media. For example, the articles may comprise a CD ROM having computer instructions for comparing gene expression profiles of the portfolios of genes described above. The articles may also have gene expression profiles digitally recorded therein so that they may be compared with gene expression data from patient samples. Alternatively, the profiles can be recorded in different representational format. A graphical recordation is one such format. Clustering algorithms such as those incorporated in "DISCOVERY" and "INFER" software from Partek, Inc. mentioned above can best assist in the visualization of such data.

Different types of articles of manufacture according to the invention are media or formatted assays used to reveal gene expression profiles. These can comprise, for example, microarrays in which sequence complements or probes are affixed to a matrix to which the sequences indicative of the genes of interest combine creating a readable determinant of their presence. Alternatively, articles according to the invention can be fashioned into reagent kits for conducting hybridization, amplification, and signal generation indicative of the level of expression of the genes of interest for predicting GvHD or TRM.

The following examples are provided to illustrate but not limit the claimed invention. All references cited herein are hereby incorporated herein by reference.

### Example 1

### I. Biomarker Candidate 1: peripheral blood dendritic cell subsets: (Disease-predictive biomarker, mechanism of action (MOA) biomarker, biomarker of treatment efficacy)

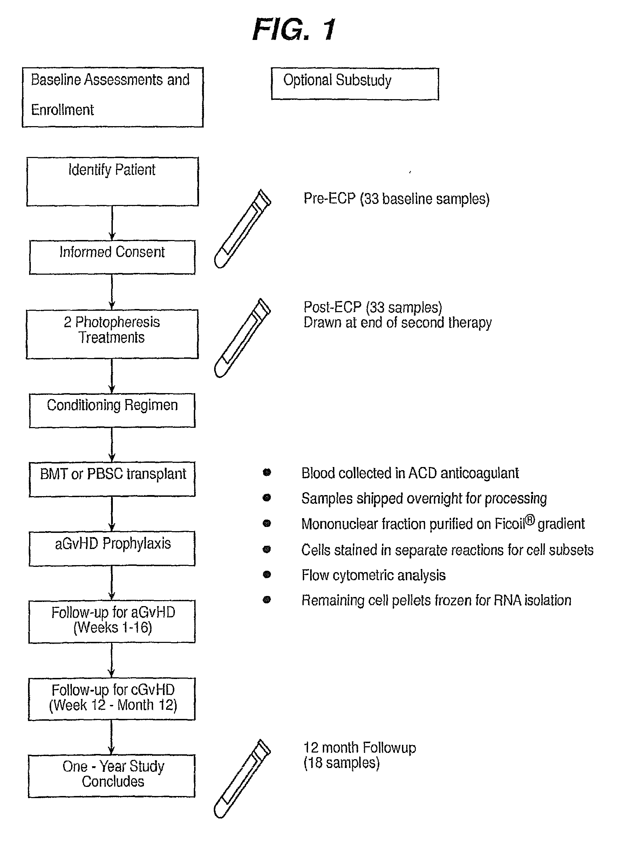

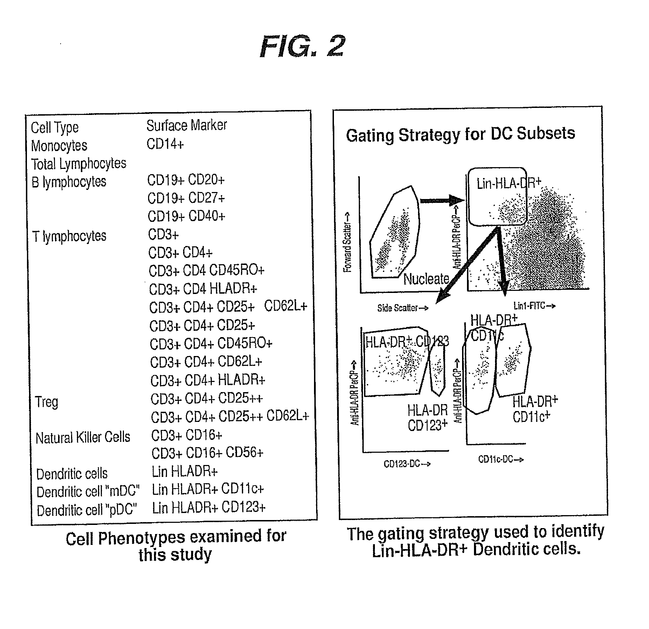

Flow cytometric analysis of sub-study samples from a GvHD prevention trial showed a statistically significant difference in the proportion of both CD11c+ and CD11c-dendritic cells (lin-/HLADR+ mononuclear cells, expressed as a fraction of mononuclear cells) in the peripheral blood of patients who suffered GvHD of grade two, three, or four compared to patients who developed no or only mild GvHD ("No GvHD," grade zero or one). The results are shown in Tables 1 and 2. Figure 1 outlines the trial and Figure 2 shows cytometric phenotyping of PBMC.

**Table 1**

| | | %HLA-DR+/CD11C+ | | %HLA-DR+/CD11C- | |
|---|---|---|---|---|---|
| Baseline | GvHD | 23.7 ± 21.00 | P=0.001 | 73.1 ± 22.11 | P=0.002 |
| | No GvHD | 45.6 ± 11.81 | | 51.1 ± 12.20 | |
| Post ECP | GvHD | 34.5 ± 24.91 | P=0.011 | 63.2 ± 25.30 | P=0.008 |
| | No GvHD | 53.7 ± 11.63 | | 43.1 ± 10.51 | |

We examined blood from a subset of 32 ECP-treated patients to identify peripheral blood cell surface markers that might predict GvHD and TRM. Samples were drawn immediately prior to ECP (at baseline) and immediately after ECP, but prior to myeloablative conditioning, then assayed by flow cytometry. Cytometry data were grouped and modeled to assess their predictive accuracy alone or in combination with clinical laboratory values. Logistic regression showed that markers for specific DC subsets present prior to myeloablation were the best predictors of outcomes. The likelihood of grade II-IV GvHD (aGvHD) increases when baseline lin- HLA-DR⁺ CD11c⁺ myeloid cells make up a smaller proportion of circulating Lin- HLA-DR⁺ cells with a predictive accuracy, reflected in the area under the receiver-operator curve (ROC) of 0.83. The best predictive model for TRM was a lower absolute abundance of circulating lin- HLA-DR⁺ CD123⁺ plasmacytoid DC at baseline (ROC=0.86). No additional predictive power arose with respect to aGvHD or TRM after including laboratory values. However, models that combined certain clinical laboratory results and demographic factors also predicted these clinical outcomes, and offer a possible alternative to complex cytometric assays. The best such model included baseline measurements of BUN/Creatinine ratio, serum albumin, and the match/relatedness of the graft donor and recipient (ROC=0.82, n=59) for TRM, while baseline neutrophil counts were most predictive of aGvHD (ROC=0.69, n=60). In summary, we have identified biomarkers including host DC subsets, readily measured clinical tests, and demographic factors that, at least in patients receiving ECP, are present before conditioning and can predict outcomes.

When absolute DC cell counts are considered, it becomes apparent that the relative contribution of CD11c+ and CD11 c- DC to a given population of peripheral blood cells can be predictive of the eventual development of GvHD in that patient. Specifically, the ratio of CD11c+ to CD11c- DC cells in a given patient sample deviates from around one in patients who do not subsequently develop severe (grade 2 to 4) GvHD to as low as 0.006 in patients who later develop severe GvHD.

Patterns in the data illustrate the ruggedness of this biomarker. Although the absolute counts shown below are small on a per microliter basis, the results are reproducible in terms of absolute count and ratio when considering counts of the related CD123+/- populations. This comparison shows a reasonable duplication in the marker (-) absolute counts and a strong relationship between CD123- and CD11c- populations. Indeed, the Lin-/HLA-DR+/CD11c- and Lin-/HLA-DR+/CD123- populations are highly correlated in both pre- and post-ECP samples (Pearson correlation coefficients r=0.95 and 0.985, respectively) suggesting these measures may represent the same cell population. In addition, these absolute counts are in line with previously reported DC counts in normal and GvHD populations. Our research is unique in the fact that it examines CD11c- and CD123- populations, while most studies examine the antigen positive, more mature populations of DC. We have thus identified a new population of DC linked to a clinical outcome.

**Table 2**

| Pre-ECP | DC absolute values | | | | | |
|---|---|---|---|---|---|---|
| Grade | HLA- | HLA- | HLA- | HLA- | Abs. | |
| GvHD | DR+/CD123+ | DR+/C123- | DR+1CD11c+ | DR+1CD11c- | Lymphs | WBC |
| 0 | 7 | 15 | 10 | 12 | 670 | 3300 |
| 0 | 5 | 12 | 9 | 8 | 401 | 2400 |
| 0 | 1 | 8 | 3 | 6 | 383 | 1200 |
| 0 | 2 | 15 | 9 | 8 | 947 | 3200 |
| 0 | 4 | 11 | 6 | 8 | 847 | 5800 |
| 0 | 3 | 4 | 3 | 4 | 252 | 3500 |
| 0 | 3 | 6 | 5 | 4 | 462 | 1600 |
| 0 | 5 | 19 | 16 | 6 | 1290 | 2300 |
| 0 | 0 | 2 | 1 | 1 | 597 | 3000 |
| 0 | 5 | 5 | 3 | 7 | 578 | 5300 |
| 0 | 3 | 17 | 15 | 5 | 672 | 4700 |
| 1 | 5 | 18 | 9 | 14 | 984 | 4600 |
| 1 | 4 | 8 | 6 | 6 | 585 | 3800 |
| 1 | 5 | 7 | 5 | 7 | 942 | 2700 |
| 1 | 1 | 103 | 44 | 59 | 1643 | 8300 |
| 2 | 7 | 19 | 11 | 15 | 332 | 2200 |
| 2 | 4 | 34 | 13 | 25 | 492 | 6000 |
| 2 | 31 | 27 | 7 | 50 | 1085 | 2700 |
| 2 | 2 | 14 | 2 | 14 | 2845 | 10900 |
| 2 | 1 | 6 | 5 | 2 | 1502 | 3500 |
| 2 | 0 | 267 | 7 | 260 | 1288 | 3700 |
| 2 | 3 | 11 | 4 | 9 | 832 | 3300 |
| 3 | 0 | 2 | 1 | 1 | 1140 | 3700 |
| 3 | 1 | 4 | 1 | 3 | 499 | 5200 |
| 3 | 2 | 7 | 3 | 5 | 779 | 4100 |
| 3 | 1 | 487 | 3 | 485 | 1705 | 3500 |
| 3 | 163 | 25 | 4 | 187 | 2415 | 11500 |
| 4 | 0 | 2 | 0 | 2 | 274 | 11900 |
| 4 | 1 | 29 | 5 | 23 | 611 | 8600 |
| 4 | 0 | 4 | 3 | 1 | 281 | 2300 |
| 4 | 2 | 24 | 3 | 23 | 422 | 1500 |
| 4 | 1 | 9 | 3 | 8 | 352 | 4000 |

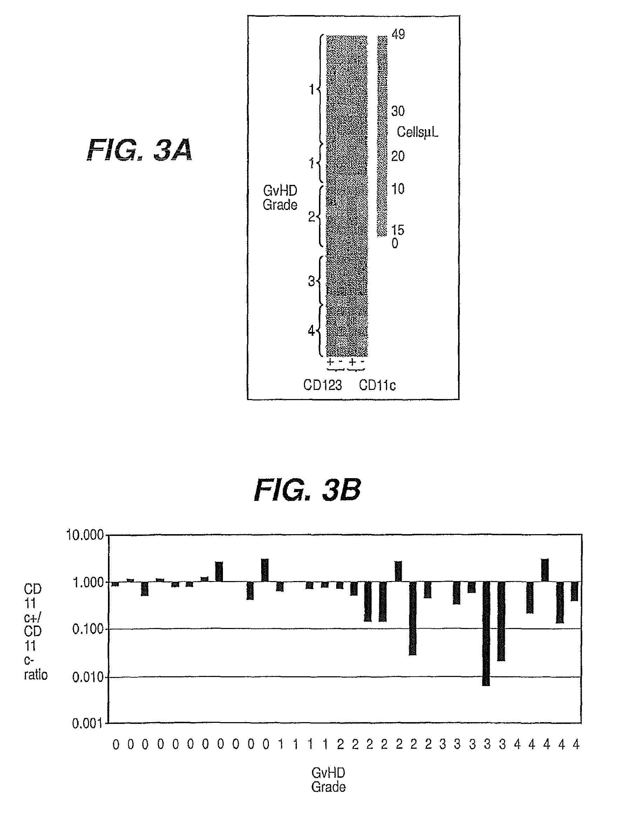

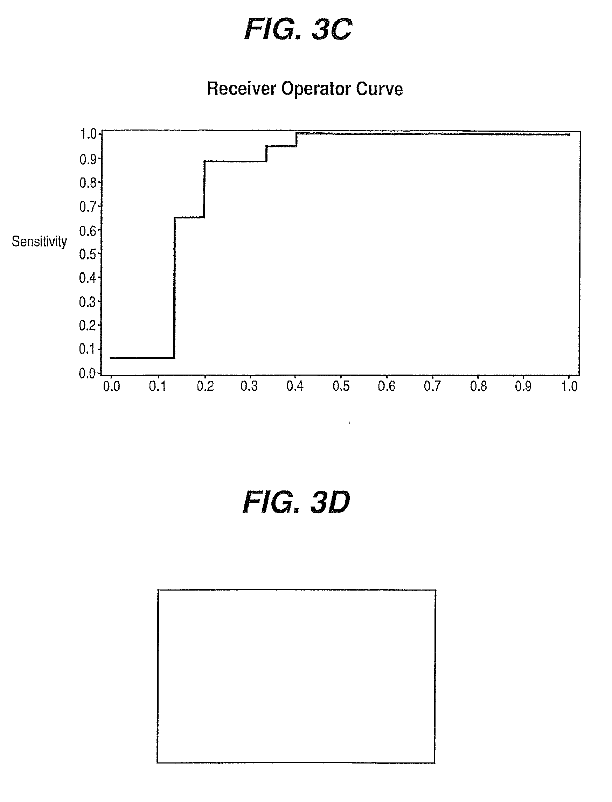

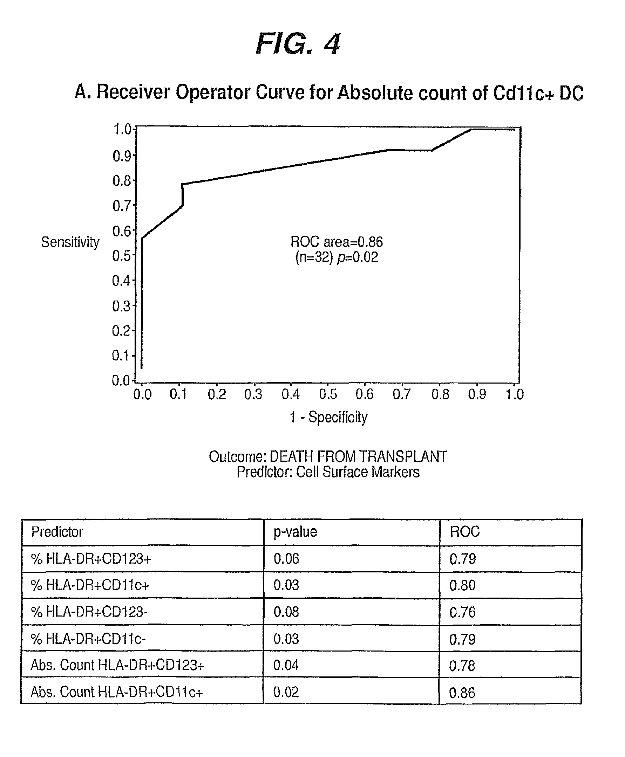

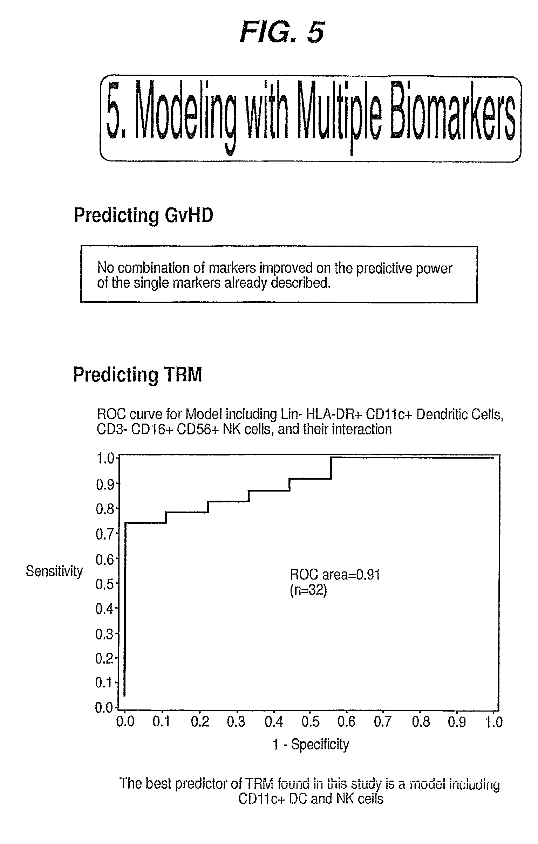

A plot of the ratio of Cd11c+ to CD11c- DC versus GvHD grade reveals a correlation between the ratio and the eventual severity of GvHD in a patient. Figure 3. Figures 4-6 show DC profiles predictive of TRM.

Other patterns include a propensity for this ratio to shift towards the positive after ECP treatment, and for the patients with a pre-BMT ratio closer to one having lower mortality (2 deaths in 0-1 vs. five in the 2-4 group). Neither the variability of the measurement nor the relationship of the donor to the recipient appear to correlate with GvHD grade, further suggesting that recipient-specific characteristics drive the propensity towards GvHD.

DC1/DC2 ratios and the relative proportion of immature DC have been proposed as prognostic indicators of disease progression and treatment outcome in numerous relevant conditions including Sezary syndrome, solid organ and bone marrow transplant including GvHD, solid tumors, atopic dermatitis, inflammatory bowel disease, and systemic viral infection.

The number of antigen negative DC correlates well with activated B cell populations. The number of CD11c- and likely the number of CD123- DC correlates with GD19+/CD27+ and CD19+/CD40+ (r=0.91 and 0.8, respectively) and less so with the total CD19+/CD20+B cell count (r=0.64) across all substudy participants. These data show that the absolute number of CD11c-/CD123- DC in the circulation is reflective of the degree of activation of B cells, a relevant consideration in GvHD.

### Example 2

### II. Biomarker candidates 2 and 3, Serum GGT and LDH (biomarkers for disease-prediction, MOA, treatment efficacy)

Using the CD11c+/CD11c- ratio, central laboratory data were examined for correlates of both the DC subset ratio and GvHD severity. Two new correlates emerged from the pre-transplant samples, elevated serum GGT and LDH. Elevation in these markers, along with significantly depressed platelet counts, allow the discrimination of individuals who later suffered high grade GvHD from those with grade zero and one GvHD in both the baseline and post-ECP samples.

Given its derivation from the CD11c ratio, these central laboratory markers meet all the criteria described for the flow cytometric marker, but have the added advantage of being readily measured and interpreted. GGT is widely used as a marker for liver damage in cirrhosis and other hepatic disorders, and LDH is typically considered a measure of cell lysis. Given that these markers are elevated in pre-transplant patients, and seem to correlate with a previously identified risk factor for GvHD (low platelet count), they may provide an additional advantage over the flow cytometric measure described above.

### Enablement of GvHD diagnostics:

These observations represent the first predictive marker or marker set for the onset and severity of acute GvHD. A qualified laboratory can readily run flow cytometry with absolute or relative quantitation of DC subsets to predict the onset of disease. Alternately, the use of monocyte and neutrophil counts in combination with other markers of susceptibility can enhance prediction and guide therapy after bone marrow transplant.

The use of these predictive cell subsets in combination with other diagnostics may allow the identification of covariate analytes (i.e. plasma or serum constituents or other biological markers) that are predictive or add to the predictive power of DC subsets. This observation and ensuing diagnostics is also relevant and generally applicable for all patients receiving bone marrow transplants.

### Example 3

Logistic regression analysis is used to identify high-risk patients (death from transplant and GVHD GRADE II-IV) given ECP, using baseline marker, laboratory and demographic variables to predict the outcome. Demographic information was collected for 62 subjects, 13 subjects had a transplant related death, and 22 subjects had grade III/IV Acute GVHD. Grade III/IV Acute GVHD was missing for one subject, this subject was not in the marker subset. The number of subjects with laboratory values varies due to missing data. The subset of patients with marker values collected has a total number of 23 patients, 9 subjects had a transplant related death and 15 subjects had grade III/IV Acute GVHD.

### Marker Values n=32

The subset of patients with baseline marker values were analyzed to determine if any of the marker values contributed to the prediction of each of the outcomes (death from transplant and GVHD GRADE II-IV). The variables provided in Tables 3 and 4 are the better predictors among all of the marker variables collected. The marker values were placed in the model as continuous variables.

**Table 3**

| **Outcome: DEATH FROM TRANSPLANT** | | | | | |
|---|---|---|---|---|---|
| | **Predictor: Markers** | | | | |
| **Model** | **n** | **Odds Ratio** | **Estimate** | **p-value*** | **ROC**** |
| p_HLAmDRsCD123s | 32 | 1.07 | 0.0715 | 0.06 | 0,79 |
| p_HLAmDRsCD11cs | 32 | 1.06 | 0.0538 | 0.03 | 0.80 |
| p_HLAmDRsCD123m | 32 | 0.94 | -0.0639 | 0.08 | 0.76 |
| p_HLAmDRsCD11cm | 32 | 0.95 | -0.0492 | 0.03 | 0.79 |
| HLAmDRsCD123s | 32 | 1.76 | 0.5673 | 0.04 | 0.78 |
| HLAmDRsCD123cs | 32 | 1.90 | 0.6397 | 0.02 | 0.86 |
| *Wald Chi-Square p-value for model coefficient | | | | | |
| ROC=Area under the receiver operator curve (Sensitivity vs. 1-Specificity) ( ) indicates the inverse Odds Ratio | | | | | |

**Table 4**

| **Outcome: GVHD GRADE II-IV** | | | | | |
|---|---|---|---|---|---|
| **Predictor: Markers** | | | | | |
| **Model** | **Estimate** | **Odds Ratio** | **Estimate *** | **P-value** | **ROC**** |
| p_HLAmDRsCD11cs | 32 | 1.08 | 0.074 | 0.01 | 0.83 |
| p_HLAmDRsCD11 cm | 32 | 0.94 | -0.067 | 0.01 | 0.80 |
| HLAmDRsCD11cs | 32 | 1.27 | 0.241 | 0.05 | 0.75 |
| *Wald Chi-Square p-value model coefficient | | | | | |
| ROC=Area under the receiver operator curve (Sensitivity vs. 1-Specificity) () indicates the inverse Odds Ratio | | | | | |

In this subset of patients, the best predictor of death due to transplant is a decrease in HLAmDRsCD123cs. The best predictor of GVHD GRADE II-IV is a decrease in p_HLAmDRsCD11 cs.

### Laboratory and Demographic Values N=62

All patients receiving ECP were analyzed to determine if any of the baseline laboratory values or demographic values contributed to the prediction of each of the outcomes. Models were selected as the 'best' model based on the significance level determined by the difference in the saturated model and the basic model's -2 log likelihood value. Results from the 'best' model are presented in Tables 5-10.

The best model for predicting death from transplant contained the variables L_BUN_CREATININE_RATIO, L_ALBUMIN_G_DL_, and Not Matched vs. Matched.

**Table 5**

| **Outcome: DEATH FROM TRANSPLANT** | | | |
|---|---|---|---|
| **Results from Best Model *** | | | |
| **Predictors in Model** | **Odds Ratio** | **Estimate** | **p-value**** |
| L_BUN_CREATININE_RATIO | 0.83 (1.20) | -0.1849 | 0.01 |
| L_ALBUMIN_G_DL_ | 8.84 | 2.1794 | 0.06 |
| Not Matched | 4.93 | 0.7983 | 0.08 |
| *AUC=0.82, n=59 | | | |
| **Wald Chi-Square p-value | | | |
| ( ) indicates the inverse Odds Ratio | | | |

The best model for predicting GVDH GRADE II-IV only contains the variable L_NEUTROPHIL_.

**Table 6**

| **Outcome: GVHD GRADE II-IV** | | | |
|---|---|---|---|
| **Results from Best Model** | | | |
| **Predictors in Model** | **Estimate** | **Odds Ratio** | **p-value*** |
| L_NEUTROPHIL_ | 0.386 | 1.039 | 0.03 |
| *AUC=0.69, n=60 (one subject was missing result for GVHD GRADE If-IV) | | | |
| **Wald Chi-Square p-value | | | |

Outcome :Labs and baseline values
The preceding tables are abstracted from the following data.

**Tables 7 and 8**

| **Marker value** | | | **Death n=9** | **None n=23** | **Total n=32** |
|---|---|---|---|---|---|
| HLAmDRsCD11cs | <=3 | n | 8.0 | 5.0 | 13.0 |
| | | % | 88.9 | 21.7 | 40.6 |
| | 3-5 | n | 1.0 | 5.0 | 6.0 |
| | | % | 11.1 | 21.7 | 18.8 |
| | 5-9 | n | 0 | 7.0 | 7.0 |
| | | % | 0 | 30.4 | 21.9 |
| | 9 < | n | 0 | 6.0 | 6.0 |
| | | % | 0 | 26.1 . | 18.8 |
| CD3mCD16sCD56s | <=110 | n | 5.0 | 4.0 | 9.0 |
| | | % | 55.6 | 17.4 | 28.1 |
| | 110-206 | n | 2.0 | 6.0 | 8.0 |
| | | % | 22.2 | 26.1 | 25.0 |
| | 206-385 | n | 1.0 | 8.0 | 9.0 |
| | | % | 11.1 | 34.8 | 28.1 |
| | >385 | n | 1.0 | 5.0 | 6.0 |
| | | % | 11.1 | 21.7 | 18.8 |

Outcome :Labs and baseline values

**Tables 9 and 10**

| Laboratory and Baseline Values | | | Death n=13 | None n=49 | Total n=62 |
|---|---|---|---|---|---|
| L_BUN_CREATININE_RATIO | <=11 | n | 3.0 | 12.0 | 15.0 |
| | | % | 23.1 | 24.5 | 24.2 |
| | 11-14 | n | 0 | 15.0 | 15.0 |
| | | % | 0 | 30.6 | 24.2 |
| | 14-19 | n | 3.0 | 14.0 | 17.0 |
| | | % | 23.1 | 28.6 | 27.4 |
| | >19 | n | 6.0 | 6.0 | 12.0 |
| | | % | 46.2 | 12.2 | 19.4 |
| | Missing | n | 1.0 | 2.0 | 3.0 |
| | | % | 7.7 | 4.1 | 4.8 |
| L_ALBUMIN_G_DL_ | <=3.9 | n | 6.0 | 10.0 | 16.0 |
| | | % | 46.2 | 20.4 | 25.8 |
| | 3.9-4.15 | n | 4.0 | 11.0 | 15.0 |
| | | % | 30.8 | 22.4 | 24.2 |
| | 4.51-4.5 | n | 2.0 | 16.0 | 18.0 |
| | | % | 15.4 | 32.7 | 29.0 |
| | >4,5 | n | 1.0 | 12.0 | 13.0 |
| | | % | 7.7 | 24.5 | 21.0 |
| Matched Vs. Not Matched | Not Matched | n | 11.0 | 22.0 | 33.0 |
| | | % | 84.6 | 44.9 | 53.2 |
| | Matched | n | 2.0 | 27.0 | 29.0 |
| | | % 1 | 5.4 | 55.1 | 46.8 |

| Outcome: GV HD grade 2-4 | | | | | |
|---|---|---|---|---|---|
| **Marker Value** | | | **GVHD n=15** | **No n=17** | **Total n=32** |
| p_HLAmDRsCD11cs | <=20.6 | n | 8.0 | 0 | 8 |
| | | % | 53.3 | 0 | 25 |
| | 20.6-35.44 | n | 4.0 | 4.0 | 8 |
| | | % | 26.7 | 23.5 | 25 |
| | 35.44-46.67 | n | 1.0 | 7.0 | 8 |
| | | % | 6.7 | 41.2 | 25 |
| | >46.67 | n | 2.0 | 6.0 | 8 |
| | | % | 13.3 | 35.3 | 25 |

| **Laboratory Value** | | | **GVHD n=22** | **Other n=39** | **Total n=61** |
|---|---|---|---|---|---|
| L_NEUTROPHIL_ | <=42.1 | n | 7.0000 | 4.0000 | 11.0000 |
| | | % | 31.8000 | 10.3000 | 18.0000 |
| | 42.1- 57.65 | n | 6.0000 | 9.0000 | 15.0000 |
| | | % | 27.3000 | 23.1000 | 24.6000 |
| | 57.65- 70.45 | n | 5.0000 | 9.0000 | 14.0000 |
| | | % | 22.7000 | 23.1000 | 23.0000 |
| | >70.45 | n | 4.0000 | 16.0000 | 20.0000 |
| | | % | 18.2000 | 41.0000 | 32.8000 |
| | Missing | n | 0 | 1.0000 | 1.0000 |
| | | % | 0 | 2.6000 | 1.6000 |

| | | | | | |
|---|---|---|---|---|---|
| Note: 1 subject missing GVHD Y/N | | | | | |

### Example 4

### prophetic

Bone marrow transplantation is a generally accepted treatment for patients suffering leukemia or other life-threatening genetic anomalies. Unfortunately, 20-50% of allogeneic hematopoietic stem cell transplant recipients succumb to graft-versus-host disease, which is a donor T cell-mediated attack on recipient tissues. Currently, prevention of GvHD relies heavily on global immunosuppression (CSA, etc) directed against T cells. Depletion of T cells from the donor population significantly diminishes the graft versus host reactions but additionally compromises engraftment, inhibits eradication of malignant cells in the recipient, and compromises reconstitution of immunity to the donor (*makes recipient susceptible* to *recurrence of leukemia*).

Due to its immunomodulatory effects, ECP has been shown to provide beneficial (life-saving) protection in several inflammatory and autoimmune diseases, including cutaneous T-cell lymphoma, scleroderma, rheumatoid arthritis, transplantation rejection, acute and chronic GvHD. Pretreatment of BMT patients with ECP is thought to work through modulation of the antigen presenting cell compartment. More specifically, dendritic cells (DCs) in both the graft and the recipient may be responsible for stimulating rejection of the allogeneic BM transplant. Maturation of DCs is an important step towards stimulation of transplant rejection. To assess whether ECP has an immunomodulatory effect on the immune cells (T cells, NK cells, and DCs), this substudy are undertaken to monitor phenotypic changes in the immune cell compartment. Blood samples are collected from each patient prior to and subsequent to photopheresis but prior to total body irradiation (TBI). A third blood sample are collected one year after receipt of transplant.

### Study Objective

To study the immunomodulatory effect of ECP with UVADEX® on the dendritic cell and T cell compartments in the peripheral blood of patients receiving standard myeloablative conditioning regimen historically compared to the standard myeloablative conditioning regimen alone on the incidence of acute and chronic GvHD in patients undergoing allogeneic sibling or unrelated BMT or peripheral blood stem cell transplant (PBSCT) for treatment of hematologic malignancies.

### Study Design and Sample Size

This multi-center substudy determines the immunomodulatory effects of ECP with UVADEX^{®} on peripheral blood dendritic cell, T cell, and NK cell compartments in patients that receive ECP followed by a myeloablative conditioning regimen of cyclophosphamide and TBI immediately prior to bone marrow transplantation. Only patients that match the inclusion criteria for the pGvHD trial and give informed consent to this substudy are included. Sample size is therefore dependent on the number of patients enrolled in the pGvHD trial that provide informed consent.

Approximately 5-6 centers participate in the prevention GvHD study. Approximately 10 patients per center receive ECP for a total of 50-60 patients enrolled into the prevention GvHD study and approximately 20-30 patients are expected to give informed consent to participation in this clinical substudy. Patients participate in this substudy for 365 days.

### Specific Explanation of Sub Study Procedures

### Sampling time

In order to perform this sub-study, a total of 3 blood samples per patient are collected at the following time-points:
◆ Sample PT1: drawn on Day -21 to Day -10 prior to ECP treatments
◆ Sample PT2: drawn on Day 7 post-transplant receipt
◆ Sample PT3: drawn on Day 365 post-transplant receipt

Patient blood is collected in heparinized vacutainer tubes labeled with patient number, visit number, date, and exact time of blood draw. The blood samples are shipped via overnight delivery to Esoterix, Inc. (Tennessee). White blood cells are separated from whole blood via Ficoll gradients. Mononuclear cells are reacted with fluorescently-labeled antibodies specific for various lineage, activation, and differentiation markers. The panels utilized to immunophenotype patient blood samples will include but not be limited to T cell, NK cell, and dendritic cell specific antibodies. Analysis of antigen expression levels are performed by flow cytometry on a Becton Dickinson FACScan.

### Technical procedure

At each sampling point, an 8ml blood sample are collected from a forearm vein via venipuncture into glass tubes containing sodium heparin. Samples are mixed by inversion and stored at room temperature prior to packaging into provided shipping containers.

### Statistical Methods

Statistical testing are performed the resulting data to determine if correlates can be made between changes in DC, T cell, or NK phenotypes and disease or treatment outcome.

### Example 5

### miRNA expression profile

DCs were obtained as described above and miRNA was obtained and analyzed as previously described. Keene et al. (2006) RIP-Chip: the isolation and identification of mRNAs, microRNAs and protein components of ribonucleoprotein complexes from cell extracts Nature Protocols 1:302-307. The results obtained are shown in Table 11.

| **Column ID** | **Mean (Pre_ECP* no_gvhd)** | **Mean(Post_ECP* no_gvhd)** | **Mean(Pre_ECP* severe)** | **Mean(Post_ECP *severe)** |
|---|---|---|---|---|
| hsa_miR_223 | **12.70** | **11.91** | **11.60** | **13.79** |
| hsa_miR_15b | **9.76** | **10.55** | **12.88** | **10.68** |
| hsa_miR_486 | **9.64** | **10.59** | **12.40** | **8.97** |
| hsa_miR_185 | **8.70** | **10.38** | **11.94** | **9.08** |
| hsa_miR_23a | **9.62** | **9.16** | **9.32** | **11.58** |
| hsa_miR_106b | **7.86** | **11.15** | **9.93** | **8.81** |
| hsa_miR_92 | **7.16** | **8.29** | **10.39** | **7.92** |
| hsa_miR_18a | **6.69** | **9.14** | **7.99** | **6.88** |
| hsa_miR_194 | **6.81** | **7.45** | **9.04** | **6.46** |
| mo miR_363_3p | **6.60** | **7.61** | **8.62** | **6.28** |
| hsa_miR_363 | **6.69** | **7.61** | **8.49** | **6.14** |
| hsa_miR_182 | **6.49** | **8.13** | **8.36** | **5.03** |
| hsa_miR_27a | **6.79** | **5.96** | **5.34** | **8.21** |
| hsa_miR_145 | **6.48** | **5.71** | **5.01** | **8.13** |
| hsa_miR_140 | **7.77** | **5.09** | **3.96** | **6.00** |
| hsa_miR_130a | **3.96** | **5.22** | **7.81** | **5.66** |
| ambi_miR_11541 | **4.77** | **5.47** | **6.78** | **4.50** |
| ambi_miR_3998 | **4.93** | **4.04** | **4.96** | **7.51** |
| hsa_miR_143 | **5.02** | **3.74** | **3.91** | **7.25** |
| hsa_miR_489 | **2.47** | **2.47** | **10.60** | **3.04** |
| hsa_miR_101 | **3.06** | **4.44** | **6.50** | **3.75** |
| hsa_miR_151 | **2.25** | **4.99** | **6.06** | **4.24** |

### NUMBERED EMBODIMENTS:

1. A method of predicting the onset and/or severity of graft versus host disease (GvHD) in a patient comprising the steps of
   a. obtaining a sample containing dendritic cells (DCs) from the patient;
   b. measuring Biomarkers associated with DC surface CD11c; and
   c. determining the ratio between CD11c+ and CD11c- cells
   wherein a ratio of less than about one is indicative of the occurrence of GvHD.
2. The method according to embodiment 1 wherein the sample is obtained after treatment of the patient with autologous apoptotic cells.
3. The method according to embodiment 2 wherein the cells are obtained by treating the patient with extracorporeal photophoresis (ECP).
4. A method of predicting the onset and/or severity of graft versus host disease (GvHD) in a patient comprising the steps of
   a. obtaining a serum sample from the patient;
   b. measuring Biomarkers associated with γ-glutamyl transferase (GGT) and lactate dehydrogenase (LDH); and
   c. determining whether the levels of these Biomarkers are elevated
   wherein an elevation of these Biomarkers prior to transplantation is indicative of the occurrence of GvHD.
5. A method of predicting the onset and/or severity of graft versus host disease (GvHD) in a patient comprising the steps of
   a. obtaining a sample containing neutrophils from the patient;
   b. measuring the number of neutrophils in a known volume; and
   wherein a reduction of neutrophils prior to transplantation is indicative of the occurrence of GvHD.
6. A method of providing direction of therapy by determining the likelihood of the occurrence of GvHD according to embodiment 1, 4 or 5 and identifying the appropriate treatment therefor.
7. A method of providing a prognosis by determining the likelihood of the occurrence of GvHD according to embodiment 1, 4 or 5 and identifying the corresponding prognosis therefor.
8. A kit for conducting an assay according to embodiment 1, 4 or 5 comprising:
   Biomarker detection reagents.
9. A microarray or gene chip for performing the method of one of embodiment 1, 4 or 5.
10. A method of predicting the onset and/or severity of transplant related mortality (TRM) in a patient comprising the steps of
   a. obtaining a sample containing dendritic cells (DCs) from the patient;
   b. measuring Biomarkers associated with plasmacytoid DC surface lin, HLA-DR and CD123; and
   wherein a lower absolute abundance of lin-HLA-DR⁺CD123⁺ plasmacytoid DCs at baseline is indicative of the occurrence of TRM.
11. A method of predicting the onset and/or severity of transplant related mortality (TRM) in a patient comprising the steps of
   a. obtaining a baseline measurement of serum γ-glutamyl transferase and lactate dehydrogenase
   wherein the elevation of these Biomarkers prior to transplantation is indicative of the occurrence of TRM.
12. A method of providing direction of therapy by determining the likelihood of the occurrence of TRM according to embodiment 10 or 11 and identifying the appropriate treatment therefor.
13. A method of providing a prognosis by determining the likelihood of the occurrence of TRM according to embodiment 10 or 11 and identifying the corresponding prognosis therefor.
14. A kit for conducting an assay according to embodiment 10 or 11 comprising:
   Biomarker detection reagents.
15. A microarray or gene chip for performing the method of embodiment 10 or 11.

## Claims

1. A method of predicting the onset and/or severity of graft versus host disease (GvHD) in a patient comprising the steps of
a. obtaining a serum sample from the patient;
b. measuring Biomarkers associated with γ-glutamyl transferase (GGT) and lactate dehydrogenase (LDH); and
c. determining whether the levels of these Biomarkers are elevated
wherein an elevation of these Biomarkers prior to transplantation is indicative of the occurrence of GvHD.

2. A method of providing direction of therapy by determining the likelihood of the occurrence of GvHD according to claim 1 and identifying the appropriate treatment therefor.

3. A method of providing a prognosis by determining the likelihood of the occurrence of GvHD according to claim 1 and identifying the corresponding prognosis therefor.

4. A kit for conducting an assay according to claim 1 comprising: Biomarker detection reagents.

5. A microarray or gene chip for performing the method of one of claim 1.
